# EUROPEAN PATENT APPLICATION

(11) **EP 2 070 543 A1**
(43) Date of publication of application: **17.06.2009**
(21) Application number: 07122971.0
(22) Date of filing: 12.12.2007
(51) Int. Cl.: A61K 35/64

(54) **High-efficiency procedure for preparing standarized hydroalcoholic propolis extract**

(71) Applicant: Bios Line S.p.a., 35020 Ponte S. Nicolo (PD) (IT)
(72) Inventor: Tramonti, Paolo, 35020, PONTE S.NICOLO'(PD) (IT)
(74) Representative: Vinci, Marcello

(57) **Abstract**

A procedure for the preparation of a hydro-alcoholic propolis extract by maceration in a bath of a hydro-alcoholic solution, facilitated by an ultrasound treatment, comprising the following stages: refrigeration of the raw propolis at a temperature below 0°C, cold grinding of said cooled raw propolis to obtain a non-adhesive powder, extraction of the active ingredients by maceration in a hydro-alcoholic solution in a thermostat-controlled bath, facilitated by an ultrasound treatment, centrifugation and filtering of the extract to remove any residue to obtain a clarified propolis extract.

## Description

The present patent relates to procedures for preparing hydro-alcoholic extracts and particularly concerns a new high-efficiency procedure for extracting active ingredients with an antioxidant, antibacterial, antifungal, anti-viral and estrogenic activity occurring in propolis (bee glue), and particularly in a type of red propolis originating from Brazil and known as *Própolis vermelha*.

### Tropical red propolis, chemical composition and biological effects

It is well known that propolis is a plant substance that originates from the resins contained in the shoots, leaves and bark of various plant species. This resinous plant exudate is collected by bees and the digested product is mixed with beeswax to obtain propolis as an end product.

Bees use propolis as an all-purpose substance for filling cracks in the beehive, modelling its walls, preventing water infiltration and draughts, but also to protect the beehive against the invasion of other insects and micro-organisms. So it is not only building material, it also represent a fundamental means of defence for the bees.

In recent years, propolis has become very popular as an ingredient in food supplements and for the treatment and prevention of various diseases. Its wide range of biological effects, its use as a health food and as a remedy in traditional medicine, have justified the renewed scientific interest focusing on propolis.

The defensive role of propolis in the beehive gives this natural matrix intrinsic biological features.

The chemical composition of propolis depends strictly on the vegetation growing in the place where it is collected and that is why it is difficult to establish any unequivocal classification of the chemical substances contained in propolis or its therapeutic properties. At the same time, it is because of these considerable qualitative and quantitative chemical differences depending on its geographical distribution that diversify and characterise propolis, making it chemically and biologically unique.

Propolis consequently has a very rich and complex chemical composition strictly related to the plant source from which it derives. In temperate climates, this plant source is represented by the genus *Populus* and the resulting bee glue has a relatively constant chemical composition from the qualitative standpoint.

In tropical regions, where the *Populus* species is not widespread, the bees (or *Apis mellifera*) use other plants to produce propolis so the chemical composition of tropical propolis is different and tends to vary considerably, given the abundance and diversity of the local flora. This situation adds to the potential value of tropical propolis from both the biological and the chemical standpoints, because it represents a rich source of new and unusual natural substances with a wide range of applications.

*Própolis vermelha* is a red propolis originating from Brazil, characterised by an unusual chemical composition: its principal ingredients belong to the class of the isoflavonoids (i.e. isoflavones, isoflavanes and pterocarpanes), and to the class of isoprenylated benzophenones.

The molecules contained in this type of propolis are the phenolic compounds isoliquiritigenin and liquiritigenin, the isoflavones formononetin and biochanin A, the isoflavanes vestitol, 7-O-methyl-vestitol and neovestitol, the pterocarpanes medicarpine, homopterocarpine, vesticarpane, 3,8-dihydroxy-9-methoxy-pterocarpane and 3-hydroxy-8,9-dimethoxy-pterocarpane, and the isoprenylated benzophenones guttiferone E and/or xanthochymol, guttiferone A and nemorosone.

The isoflavonoids are the "*markers*" or chemical compounds characteristic of red propolis (*Piccinelli*, *2005; 2007*). This class of natural compounds has recently attracted considerable interest in the scientific world because of the interesting biological effects they have revealed.

In particular, the isoflavones represent the main class of plant estrogens, i.e. substances that structurally and functionally mimic human endogenous estrogens, that have proved beneficial to human health; they have an important role in the prevention of cancer and cardiovascular diseases, and in the treatment of the symptoms of menopause and osteoporosis (*Setchell, 1998; Adlercreutz, 2002).*

The isoflavanes have also been identified as metabolites of the plant estrogens in the human diet, and some have shown a stronger estrogenic activity than their precursors. No estrogenic activity has been reported for the pterocarpanes, though they have revealed other interesting biological effects, particularly consisting in the demonstration that phyto-alexins have a strong anti-mycotic (*Perrin, 1969*) and antibacterial (*Donnelly, 1995)* activity.

Some pterocarpanes have shown an inhibitory effect on the reverse transcriptase of the HIV-1 virus (*Engler*, *1993*) and an inhibitory effect on COX-2 and anti-inflammatory activity in vivo (*Selvam*, *2004*) has also recently been reported.

The isoprenylated benzophenones are secondary metabolites characteristic of the *Clusiaceae* family, plants that are very common in Brazil and that have been identified among the sources of the propolis coming from tropical areas. Current interest in the isoprenylated benzophenones with a bicyclo[3.3.1]nonan nucleus is due to the biological actions they have revealed (*Cuesta-Rubio, 2005),* the most interesting of which are certainly the cytoprotective effect against the HIV-1 virus and their antimicrobial and cytotoxic activities.

For instance, guttiferones and nemorosone have an anti-HIV effect with an IC 50 = 1-10 µg/ml (*Gustafson*, *1992*, *Piccinelli 2005);* garcinol has recently aroused considerable interest for its remarkable beneficial properties, such as an antioxidant effect (*Yamaguchi*, *2000a e 2000b*), a chemo-preventive action in neoplastic disease, and its capacity to induce apoptosis in mutated cells; a combination of xanthochymol with guttiferone E has shown a Paclitaxel-like inhibitory effect on microtubule disassembly (*Roux, 2000*). A strong cytotoxic activity against certain tumour cell lines, such as human cervical carcinoma (HeLa), human laryngeal carcinoma (HeP-2), prostate carcinoma (PC-3) and central nervous system carcinoma (U251), has been identified for nemorosone too (*Cuesta-Rubio*, *2005*).

Very often, the biological effects of propolis are attributed to the presence of phenolic compounds, but bee glues with different chemical compositions also have similar properties (*Marcucci*, *1995; Burdock, 1998*). Given the remarkable abundance and variety of the flora in the tropical regions, the propolis coming from these areas represents a rich source of new and unusual natural substances with a wide range of applications.

### Extraction

The solid-liquid extraction technique currently most often used to extract propolis is by maceration, which is the most straightforward and economical extraction method. Maceration alone demands lengthy extraction times, however, and very often the matrix is only partially extracted, in which case we speak of incomplete extraction.

Using the known maceration technique, the extraction process takes place as a result of diffusion and osmotic phenomena, which depend heavily on temperature. Extraction efficiency can consequently be improved and/or the process accelerated by increasing the temperature or performing a dynamic maceration.

It is common knowledge that these drawbacks of maceration, i.e. long extraction times and incomplete extraction, can be partially overcome by facilitating the maceration process using ultrasound or microwaves, which are capable of increasing the kinetic energy of the molecules within the solid matrix.

Though it demands the use of more costly equipment, this type of extraction technique reduces the extraction times and increases the output.

The main goals of the extraction of propolis consist in maximising the extraction of the active ingredients and the removal of the inert substances (mainly waxes) that contribute little or nothing to the efficacy of the extracts - quite the reverse, in fact, they very often interfere with its stability and subsequent conversion into hydro-soluble and/or hydro-dispersible products. In fact, most of these waxes are insoluble in alcohol and their solubility further diminishes in hydro-alcoholic mixtures. Conversely, the active ingredients of propolis are perfectly soluble in alcoholic solvents, while its solubility in hydro-alcoholic mixtures depends mainly on the nature of its chemical constituents. A small proportion of water generally optimises the quantity of active ingredients obtained because it does not significantly influence their solubility while it does considerably reduce the percentage of waxes. Generally speaking, the solvent used to prepare propolis extracts contains 60-80% ethanol.

It is well known that the procedure for extracting the active ingredients of propolis first involves grinding the propolis to obtain fine granules, approximately 3 mm in diameter (up to a maximum of 5 mm).

After grinding, the propolis is added to a mixture of ethanol and water, in a volume/volume ratio of 87:13.

The extraction process, which takes approximately 25 minutes, is conducted at a controlled temperature coming between 0° and 20°C, and it is facilitated by an ultrasound treatment at a frequency between 18 and 25 kHz.

The propolis extract is decanted and filtered to separate it from the residual wax, then compressed in a vacuum, e.g. at a pressure between 1471 and 490 Pa, to obtain the required percentage of dry matter, up to a maximum of 80%.

This procedure has some drawbacks, however, the first of which lies in the fact that when the grinding process is conducted using the conventional methods, it can induce an increase in the temperature of the propolis that may damage a part of its temperature-sensitive active ingredients already in the initial stages of the extraction procedure.

Another drawback lies in that granules at least 3 mm in diameter can be obtained using the grinding process according to the known technique, but smaller dimensions cannot be attempted to avoid further overheating the matrix. The contact surface between the granules and the solvent is consequently limited and cannot be further increased, with the resulting drawback that the efficiency of the extraction process is also limited.

To overcome the above-mentioned drawbacks, a new high-efficiency procedure has been studied and implemented for extraction the active ingredients with an antioxidant, antibacterial, antifungal, anti-viral and estrogenic activity contained in propolis, and particularly in a type of red propolis originating from Brazil and known as *Própolis vermelha.*

In particular, the present patent concerns the development of a procedure for the production, with the aid of ultrasound, of a standardised hydro-alcoholic extract with a high content of active ingredients from red propolis or *Própolis vermelha,* which is characterised by an unusual chemical composition that reflects the biodiversity of the flora of the tropical regions.

The present procedure for the extraction of the active ingredients of *Própolis vermelha* using ultrasound involves the following stages:
1. converting the original material, i.e. said raw propolis, into a powder that is homogeneous from the point of view of its chemical composition by grinding in cooled conditions, after storage for 24 hours in the freezer, followed by sieving to obtain a non-adhesive powder with a homogeneous particle size distribution of approximately 500 µm;
2. obtaining propolis extract from said homogeneous material by maceration in a hydro-alcoholic ethanol solution, facilitated by an ultrasound treatment at a frequency of approximately 59 kHz for 2 hours in a thermostat-controlled bath at 25° C. The drug/solvent ratio and the optimal solvent to use in the extraction process are 20% of propolis in 80% of hydro-alcoholic ethanol solution;
3. centrifuging and filtering the extract to remove any residual material and obtain a clarified propolis extract;
4. determining the content of the most abundant and representative constituents of *Própolis vermelha -* such as guttiferone E for the class of the isoprenylated benzophenones, medicarpine for the pterocarpanes and methyl vestitol for the isoflavanes - by means of a specifically developed HPLC-DAD-MS quantitative analytical method.
Thus, by comparison with the traditional methods for preparing propolis extracts, the present new procedure is distinguishable because of the following novelties and advantages:
- the extraction process is performed on a material that is homogenous from the chemical standpoint so as to ensure a constant titre of the end product; a hydro-alcoholic extract is obtained that is standardised in terms of its content of active ingredients of propolis, and of *Própolis vermelha* in particular;
- extraction with a hydro-alcoholic solution of 80% ethanol ensures a fairly limited solubility of the waxes, thereby obtaining a clear solution along with a complete extraction of all the other, active ingredients;
- the extraction procedure is highly efficient: the ultrasound treatment enables the complete extraction of all the bio-active substances so a much more effective propolis extract is obtained than with the known technique of maceration alone;
- the ultrasound-supported extraction process takes approximately 2 hours to complete, a much shorter time than with the known maceration techniques;
- the extraction procedure according to the new procedure demands a lower consumption of ethanol;
- the new procedure can be implemented in less space than the traditional maceration method, which demands the use of several tanks made of a rustproof material that are not necessary for the new procedure;
- the entire extraction procedure has been applied to a new type of propolis, a red propolis from Brazil called *Própolis vermelha,* which has a different chemical composition from the commonly-marketed products, and that is characteristic of the tropical regions and has numeral possible applications;
- the resulting hydro-alcoholic extract of *Própolis vermelha* is rich in active ingredients such as guttiferone E (7.7 mg/ml), medicarpine (8.0 mg/ml) and methyl vestitol (3.6 mg/ml), in much larger quantities than those achievable using the traditional extraction technique: by comparison with maceration alone, there is a 205-226% increase in the extraction efficiency of the active ingredients.
The invention is described in more detail in the following examples.

### Extract production

According to the new procedure, the raw propolis homogenisation phase involves a cold grinding process that is used to preserve the active ingredients and to obtain a non-adhesive powder with a constant particle size distribution.

For this purpose, the raw propolis is first stored in a freezer for 24 hours, at -18°C for instance, to reduce its adhesiveness and thereby enable an effective grinding action. The grinding phase can be conducted using a knife mill operated at intervals, in brief cycles, to avoid any heating of the matrix and consequent increase in its adhesiveness.

Alternatively, or in combination with said procedure, the raw propolis can be cooled to temperatures higher than -18°C and said grinding phase can be completed in a refrigerated environment, again with a view to restricting any increase in the temperature of the matrix during the grinding process.

The resulting ground propolis is sieved through a test sieve with a 500 µm mesh to obtain a powder with a homogeneous particle size distribution.

The extraction process is facilitated by an ultrasound treatment to overcome the main drawbacks of maceration alone, i.e. lengthy extraction times and incomplete extraction.

For the extraction process, the homogenised propolis powder is placed in 80% ethanol, with a drug-to-solvent ratio of 2:10, i.e. a solution containing 20% of propolis, for 2 hours in a thermostat-controlled bath with ultrasound at a frequency of 59.0 kHz and a temperature of 25° C.

The extract is subsequently centrifuged and filtered.

The resulting extract of *Própolis vermelha* has undergone quantitative analyses using HPLC-DAD-ESI/MS to determine the content of the active ingredients of this type of propolis.

Table 1 below shows the values for the composition of the 20% hydro-alcoholic extract of *Própolis vermelha,* obtained using the previously described procedure.

**Table 1 -Composition of the Própolis vermelha 20% hydro-alcoholic extract.**

| **Active ingredients** | **Content (mg/ml)** |
|---|---|
| Guttiferone E | 7.74 ± 0.44 |
| Medicarpine | 8.05 ± 0.06 |
| Methyl vestitol | 3.62 ± 0.32 |

### Applications

It is well known that plant extracts containing active ingredients with a wide range of biological effects are already available on the market. These active ingredients are used in various cosmetic, dietary, pharmaceutical and herbal medicine sectors.

The main aim of the present invention is to realise a system for the extraction of active ingredients with antioxidant, antibacterial, antifungal, anti-viral and estrogenic characteristics specifically suitable for use in the pharmaceutical, cosmetic, nutritional and zootechnical fields.

In the context of the above-stated technical aim, a particular object of the present invention is to realise an extraction system that enables a product to be obtained that offers considerable advantages, in the above-mentioned fields, as an adjuvant in conventional pharmaceutical treatments and/or as a dietary supplement, being capable both of reducing oxidative stress and of protecting the body against pathogenic micro-organisms.

One of the principal advantages of the new procedure consists in that it achieves a highly efficient extraction of the active ingredients, thanks to the particular and innovative method adopted.

First of all, as mentioned previously, the grinding is done on raw propolis previously preserved in the freezer, obtaining the dual result of reducing the adhesiveness of the material and limiting the rise in its temperature due to the grinding phase, with the advantage that the active ingredients are consequently better preserved.

The grinding is also done with a knife mill operating in short cycles, not continuously, to contain the heating of the material.

The ground propolis is sieved through a 500 µm mesh to obtain a non-adhesive powder of homogeneous particle size distribution, in order to obtain a larger specific surface area in contact with the solvent in the subsequent extraction phase and a consequently higher extraction efficiency. During the extraction process, which takes 2 hours instead of the 20-25 minutes of the known techniques, the solution undergoes an ultrasound treatment at a higher frequency than is normally used.

The frequency of the ultrasound treatment and the duration of the extraction process contribute to the achievement of a high-efficiency extraction of the active ingredients in the propolis, and particularly in the red propolis,

### Própolis vermelha.

Tables 2 and 3 below provide comparative data relating to the concentration of active ingredients in mg per 100 ml in the alcoholic extract and to the percentage titre of active ingredients in the dry residue (d.r.), obtained respectively by the extraction procedure according to the known classic technique and according to the new method applied to red propolis.

It is clear from Table 2 that the new procedure enables a much higher concentration of active ingredients to be achieved than the one obtainable with the known method, with an increase that ranges from 182% (for methyl vestitol) to 228% (for other benzophenones).

Table 3 clearly shows that the new procedure enables the preparation of an extract with a much higher percentage titre of active ingredients than is achievable using the known method, with an increase ranging from 235% (for methyl vestitol) to 270% (for guttiferone E).

**Table 2 - Red propolis: Quantity of active ingredients in the alcoholic extract**

| | **Medicarpine (mg/100 ml)** | **Methyl vestitol (mg/100ml)** | **Guttiferone E (mg/100ml)** | **Others benzophenones (mg/100ml)** |
|---|---|---|---|---|
| **Classic method** | 393 ± 13 | 198 ± 5 | 359 ± 6 | 203.5 ± 3 |
| **New procedure** | 805 ± 6 | 362 ± 32 | 774 ± 44 | 464 ± 31 |

**Table 3 - Red propolis: Percentage titre of active ingredients calculated in the dry residue**

| **Dry residue (mg/ml d.r.)** | **Medicarpine (% d.r.)** | **Methyl vestitol (% d.r.)** | **Guttiferone E (% d.r.)** | **Other benzophenones (% d.r.)** |
|---|---|---|---|---|
| **Classic method** (98 ± 4) | 4.0 | 2.0 | 3.7 | 2.1 |
| **New procedure** (77 ± 3) | 10.5 | 4.7 | 10.0 | 5.5 |

The extraction process takes place at room temperature, around 25°C, so it demands no particular operating conditions - unlike the known techniques, in which the temperature is kept low (between 0°C and 20°C) during the maceration process, which consequently demands further operating and management costs.

Another advantage of the present invention lies in that the ethanol used in the extraction process is 80% biological ethanol, i.e. in a lower concentration than the one used in the known technique, which demands the use of 87% ethanol.

Another object of the present invention is to achieve an extraction process that enables a product to be obtained - even formulated and in synergy with other active ingredients, plant extracts, vitamins and mineral salts - for use in the treatment of numerous symptoms due to situations of particular stress, various physiological times of life, climate changes, poor nutrition, influenza, minor diseases of the upper airways, inflammation of the oral cavity and pharynx, etc. It can be usefully adopted to help protect the body against certain viral infections and the harmful effects of environmental pollution, to maintain good general health, as an antibiotic treatment, and generally as a treatment for the symptoms of ageing.

Another, not necessarily last object of the present invention is to achieve an extraction process in which the particular way in which the extract is prepared is capable of ensuring the widest possible guarantee of reliability and safety in use of the resulting end product.

The above-described technical aims and objects are achieved by the new procedure for extracting active ingredients in the isoprenylated benzophenone, pterocarpane and isoflavane classes, **characterised in that** it involves their extraction from a homogenised material with the aid of ultrasound at a frequency of 59.0 kHz for 2 hours in a thermostat-controlled bath at 25°C in a hydro-alcoholic ethanol solution. The optimal drug-to-solvent ratio and proportion of solvent used in the extraction process are 20% propolis and 80% of a hydro-alcoholic ethanol solution.

Thus, with reference to the above description, the following claims are advanced.

## Claims

1. A procedure for the preparation of a hydro-alcoholic propolis extract by maceration of a hydro-alcoholic solution in a bath, facilitated by an ultrasound treatment, **characterised in that** it comprises the following stages:
- refrigeration of the raw propolis at a temperature below 0°C;
- cold grinding of said cooled raw propolis to obtain a non-adhesive powder;
- extraction of the active ingredients by maceration of a hydro-alcoholic solution in a thermostat-controlled bath facilitated by an ultrasound treatment;
- centrifugation and filtering of the extract to remove any residues in order to obtain a clarified propolis extract.

2. A procedure according to claim 1, **characterised in that** said raw propolis is preserved at a temperature of -18°C or lower for 24 hours.

3. A procedure according to claims 1, 2, **characterised in that** said cold grinding of the cooled raw propolis is completed at intervals, in short cycles, using a knife mill, to prevent any overheating and consequent increase in the adhesiveness of the material.

4. A procedure according to claims 1, 2, 3, **characterised in that** said cold grinding of the raw propolis is done to obtain a homogeneous powder.

5. A procedure according to claims 1, 2, 3, 4, **characterised in that** said propolis powder obtained from the grinding process undergoes a sieving stage.

6. A procedure according to claim 5, **characterised in that** said sieving stage is conducted using sieves with a 500 µm mesh to obtain a powder with a homogeneous particle size distribution.

7. A procedure according to claims 1, 2, 3, 4, 5, 6, **characterised in that** said extraction phase involves maceration in a hydro-alcoholic ethanol solution, facilitated by ultrasound at a frequency of 59.0 kHz, lasting 2 hours in a thermostat-controlled bath at 25° C.

8. A procedure according to claims 1, 7, **characterised in that** said extraction takes place in an 80% hydro-alcoholic ethanol solution with a drug-to-solvent ratio of 2:10.

9. A procedure according to the previous claims, **characterised in that** said clarified propolis extract undergoes quantitative analysis by HPLC-DAD-ESI/MS to determine the content of its main constituents.

10. A procedure according to the previous claims, **characterised in that** it uses raw propolis of the type called *Própolis vermelha*, or red propolis, originating from Brazil.

11. A hydro-alcoholic propolis extract containing active ingredients with antioxidant, antibacterial, antifungal, anti-viral and estrogenic characteristics that is specifically suitable for use in the pharmaceutical, cosmetic, nutritional and zootechnical fields, **characterised in that** it is obtained from propolis of the type called *Própolis vermelha,* or red propolis of Brazilian origin, according to the procedure described in one or more of the previous claims.

12. A hydro-alcoholic propolis extract containing active ingredients with antioxidant, antibacterial, antifungal, anti-viral and estrogenic characteristics that is specifically suitable for use in the pharmaceutical, cosmetic, nutritional and zootechnical fields, **characterised in that** its principal active ingredients include: guttiferone E, medicarpine and methyl vestitol.
